(19) Europäisches Patentamt
European Patent Office
Office européen des brevets

(11) Publication number: **0 202 568**
**A1**

(12) # EUROPEAN PATENT APPLICATION

(21) Application number: 86106403.8

(22) Date of filing: 12.05.86

(51) Int. Cl.[4]: A 61 K 31/165
A 61 K 31/415, A 61 K 47/00
//(A61K31/415, 31:165)

(30) Priority: 13.05.85 US 733170
13.05.85 US 733172

(43) Date of publication of application:
26.11.86 Bulletin 86/48

(84) Designated Contracting States:
AT BE CH DE FR GB IT LI LU NL SE

(71) Applicant: SYNTEX (U.S.A.) INC.
3401 Hillview Avenue
Palo Alto, California 94304(US)

(72) Inventor: Freckelton, Brian
2A Warrimoo Avenue
St. Ives, N.S.W. 2075(AU)

(74) Representative: Barz, Peter, Dr. et al,
Patentanwälte Dipl.-Ing. G. Dannenberg Dr. P. Weinhold, Dr. D. Gudel Dipl.-Ing. S. Schubert, Dr. P. Barz
Siegfriedstrasse 8
D-8000 München 40(DE)

(54) **Anthelmintic compositions.**

(57) Veterinary compositions in convenient dosage form comprising rafoxanide, a solvent selected from dimethyl isosorbide and glycofurol, optionally a nonionic surfactant, and, optionally, an anthelmintic benzimidazole derivative are disclosed.

EP 0 202 568 A1

ANTHELMINTIC COMPOSITIONS

This invention relates to veterinary compositions containing the fasciolicide rafoxanide, a solvent selected from dimethyl isosorbide and glycofurol, optionally a nonionic surfactant, and optionally an anthelmintic benzimidazole derivative of formula I

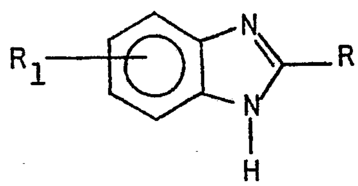

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

Example of compounds of formula I include oxfendazole, albendazole, fenbendazole, mebendazole, oxibendazole, parbendazole, flubendazole, thiabendazole, cambendazole, or cyclobendazole. These compositions are useful for treating helminthiasis either orally or by intrarumenal injection, and are particularly valuable for administration by intrarumenal injection.

Certain anthelmintic benzimidazole derivatives are known: see, e.g., U.S. Pat. Nos. 4,080,461; 4,002,640; 3,965,113; 3,929,821; 3,915,986; 3,480,642; 3,574,845; 3,017,415; 3,657,267; South African Patent No. 6,800,351; and Belgian Patent No. 793,358. Rafoxanide is a known fasciolicide; see U.S. Pat. No. 3,914,418.

Certain combinations of oxfendazole and other anthelmintics are known. See, e.g., U.S. Pat. Nos. 4,436,737 (Boray), 4,173,632 (Cruthers), 4,166,858 (Rowlands) and 4,159,337 (Rowlands). Boray discloses a combination of oxfendazole with O-(4-bromo-2-chlorophenyl)-O-ethyl-S-propylphosphothioate (profenofos). Cruthers discloses a combination of oxfendazole and 3,5-dibromo-N-(4-bromophenyl)-2-hydroxybenzamide (tribromosalan). Rowlands discloses a combination of oxfendazole and bis-(β-(4-acetamidophenoxy)ethyl)ether (diamphenethide).

Also, certain combinations of rafoxanide are known. See, e.g., U.S. Pat. No. 3,956,488 (Egerton), which discloses a combination of rafoxanide with 5-isopropoxy-

carbonylamino-2-(4-thiazolyl)benzimidazole (cambendazole) without the solvent employed in the present invention.

A new combination of veterinary agents is here disclosed. It was discovered that administration of rafoxanide with or without an anthelmintic benzimidazole derivative in a convenient dosage form, e.g., by rumen injector or oral applicator, is hindered by the extremely low solubility of each component in nearly all pharmaceutically acceptable solvents or the high viscosity of any resulting solution or suspension. The solvent used must be pharmaceutically acceptable and chemically stable, must be capable of dissolving or suspending each component, and must not have an unreasonably high viscosity so that the formulation may be administered with a rumen injector or oral applicator. The formulation of veterinary agents should also permit easy clean-up of the rumen injector or oral applicator after use. The surfactant used may enhance the activity of the formulation but must not reduce it.

It has now been discovered that the problems of the art are overcome by the combination of rafoxanide with dimethyl isosorbide or glycofurol, optionally a nonionic surfactant, and optionally a compound of formula I.

The first aspect of this invention is a composition comprising

rafoxanide;

a solvent selected from dimethyl isosorbide and glycofurol;

optionally a nonionic surfactant; and

optionally a compound of formula I

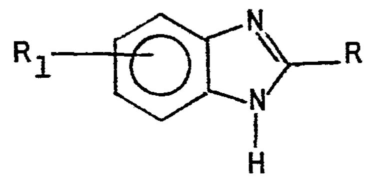

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

Another aspect of the invention is a composition comprising

rafoxanide;

a solvent selected from dimethyl isosorbide and glycofurol;

optionally a nonionic surfactant; and

optionally a compound of formula I wherein

R is $-NHCOOR_2$, where $R_2$ is lower alkyl;

$R_1$ is $-S(O)_mR_3$, -SCN, $-OR_3$, or $-Y_1(CH_2)_nY_2R_4$, where

$Y_1$ and $Y_2$ are each independently O, S, S(O), or $S(O)_2$;

$R_4$ is lower alkyl, phenyl, or naphthyl;

n is an integer from 1 to 4;

$R_3$ is lower alkyl, cycloalkyl having 3 to 7 carbon atoms, lower alkenyl or lower alkynyl, phenyl, benzyl, phenylethyl, or naphthyl; and

m is 1 or 2.

Another aspect of the invention is a composition comprising

rafoxanide;

a solvent selected from dimethyl isosorbide and glycofurol;

optionally a nonionic surfactant; and

optionally a compound of formula I or a pharmaceutically acceptable salt thereof, wherein

R is $-NHCOOR_2$ or 4-thiazolyl, where $R_2$ is alkyl, or aryl; and

$R_1$ is H, $R_3$, or $-XR_3$, where

X is O, S, -C(O)-, or -NHCOO-; and

$R_3$ is alkyl or cycloalkyl having 3 to 7 carbon atoms, aryl or aryl-alkyl, optionally substituted with halo, alkyl, hydroxy, or alkoxy.

Another aspect of the invention is an anthelmintic benzimidazole-free composition comprising rafoxanide, a solvent selected from dimethyl isosorbide and glycofurol, and optionally a nonionic surfactant.

Another aspect of the invention is an anthelmintic benzimidazole-free composition comprising rafoxanide, a solvent selected from dimethyl isosorbide and glycofurol, and a nonionic surfactant.

Another aspect of the invention is an anthelmintic benzimidazole composition comprising rafoxanide, a solvent selected from dimethyl isosorbide and glycofurol,

optionally a nonionic surfactant, and a compound of formula I

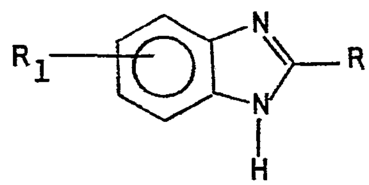

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

Another aspect of the invention is an anthelmintic benzimidazole composition comprising rafoxanide, a solvent selected from dimethyl isosorbide and glycofurol, a nonionic surfactant, and a compound of formula I

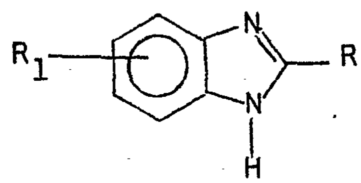

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

Another aspect of the invention is a method for treating helminth and/or fluke infections in a ruminant by administering a therapeutically effective amount of a composition described above. A preferred aspect of the invention is the method for treating helminth and fluke infections in a ruminant by administering a therapeutically effective amount of a compound of formula I and rafoxanide in a glycofurol or dimethyl isosorbide suspension optionally containing a nonionic surfactant by oral administration or intrarumenal injection, particularly by intrarumenal injection.

A more preferred aspect of the invention is the method for treating helminth and fluke infections in a ruminant by administering a therapeutically effective amount of a compound of formula I and rafoxanide in a glycofurol or dimethyl isosorbide suspension containing a nonionic surfactant by oral administration or intrarumenal injection, particularly by intrarumenal injection.

## DEFINITIONS

As used in the specification and the appended claims, unless specified to the contrary, the following terms have the meaning indicated:

The term "pharmaceutically acceptable acid addition salts" refers to salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid and the like.

The term "treatment" as used herein covers any treatment of a disease in a ruminant, and includes:

(i) inhibiting the disease, i.e., arresting its development; or

(ii) relieving the disease, i.e., causing regression of the disease.

"Oxfendazole" refers to the compound methyl [5-(phenylsulfinyl)-1H-benzimidazol-2-yl] carbamate.

The term "albendazole" refers to the compound methyl 5-(propylthio)-1H-benzimidazol-2-yl carbamate.

The term "fenbendazole" refers to the compound methyl 5-(phenylthio)-1H-benzimidazol-2-yl carbamate.

The term "mebendazole" refers to the compound methyl 5-benzoyl-1H-benzimidazol-2-yl carbamate.

The term "oxibendazole" refers to the compound methyl 5-(1-propoxy)-1H-benzimidazol-2-yl carbamate.

The term "parbendazole" refers to the compound methyl 5-(1-butyl)-1H-benzimidazol-2-yl carbamate.

The term "flubendazole" refers to the compound methyl 5-(4-fluorobenzoyl)-1H-benzimidazol-2-yl carbamate.

The term "thiabendazole" refers to the compound methyl 2-(4-thiazolyl)-1H-benzimidazole.

The term "cambendazole" refers to the compound isopropyl 2-(4-thiazolyl)-1H-benzimidazol-5-yl carbamate.

The term "cyclobendazole" refers to the compound methyl 5-cyclopropylcarbonyl-1H-benzimidazol-2-yl carbamate.

"Rafoxanide" refers to the compound N-[3-chloro-4-(4-chlorophenoxy)-phenyl]-2-hydroxy-3,5-diiodobenzamide.

"Glycofurol" refers to the compound α-(tetrahydrofuranyl)-ω-hydroxypoly-(oxy-1,2-ethanediyl). Glycofurol is available commercially from AGRAR Soc. a r.l., Rome, Italy under the name Tetraglycol. Glycofurol is a mixture of compounds of formula II (where n is an integer varying from 1 to 5) having the following characteristics: boiling point at 0.5-0.01 mmHg = 80-155°C; index of refraction = 1.459-1.464; and density at 20°C = 1.082-1.092.

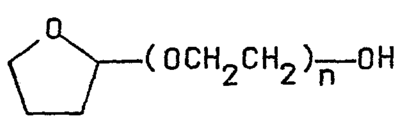

(II)

"Dimethyl isosorbide" refers to the compound 2,5-dimethoxy-1,3,4,6-dianhydrosorbitol. Please see formula III.

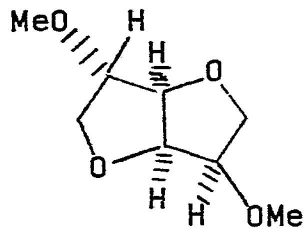

(III)

The term "nonionic surfactant" refers to those compounds in the class typified by nonoxynol, octoxynol, and nonoxinol. Compounds in this class are sold under such tradenames as Conco NI, Dowfax N, Sterox, Triton N, Conco NIX, and Triton X, with the individual member of the series indicated by numerical suffixes, for example Triton•X100.

The term "octoxynol" refers to the compounds α[4-(1,1,3,3,-tetramethylbutyl)phenyl]-ω-hydroxy-poly(oxy-1,2-ethanediyl). Octoxynol (N.F.) is a mixture of compounds of the formula

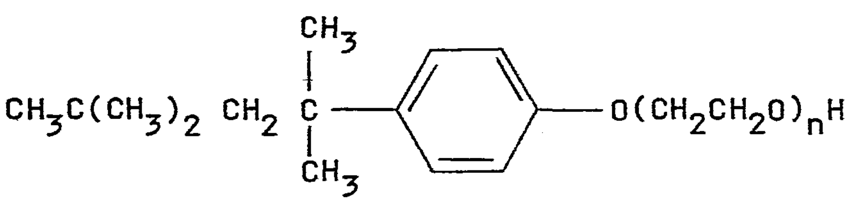

in which n ranges from 5 to 15. Triton•X100, one of the octoxynol class, is available from Rohm & Haas, U.S.A.

The term "nonoxynol" refers to the compounds α-(nonylphenyl)-ω-hydroxypoly(oxy-1,2-ethanediyl). Nonoxynol is a mixture of compounds of the formula

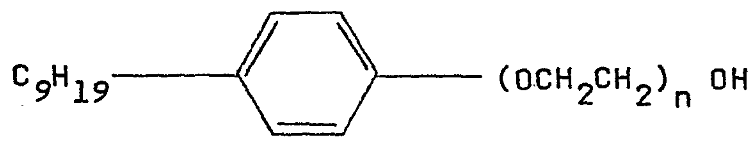

The average number of ethylene oxide units (n) per molecule is indicated by the number following nonoxynol (for example nonoxynol-15 for n = 15).

"Anthelmintic benzimidazole derivatives" refers to compounds of formula I

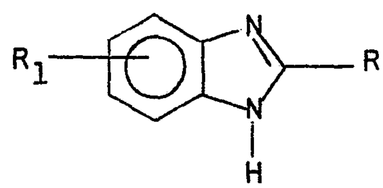

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

The term "therapeutically effective amount" of rafoxanide or an anthelmintic benzimidazole refers to an amount sufficient to effect treatment, as defined above.

The term "sufficient amount" of a solvent means an amount of solvent capable of suspending and/or dissolving all components of the composition.

The term "alkyl" refers to a straight or branched chain saturated hydrocarbon radical containing 1 to 20 carbon atoms. Typical examples of alkyls include, for example, methyl, ethyl, n-propyl, isopropyl, n-butyl, isobutyl, t-butyl, n-amyl, n-hexyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, and n-eicosyl.

The term "Lower alkyl" refers to a straight or branched chain alkyl radical of 1 to 6 carbons. Typical examples are listed under "alkyl" above.

The term "cycloalkyl" refers to a cyclic alkyl radical of 3 to 7 carbon atoms such as, for example, cyclopropyl, cyclopentyl, cyclohexyl, and the like.

The term "alkoxy" refers to a radical of the form RO-, where R is alkyl or cycloalkyl as defined above. Typical alkoxy groups include, for example, methoxy, ethoxy, t-butoxy and the like.

The term "aryl" refers to an aromatic hydrocarbon radical containing 3 to 15 carbon atoms. Typical examples include, for example, phenyl and naphthyl.

The term "halo" refers to fluoro, chloro, bromo, or iodo.

The terms "lower alkenyl" refers to an unsaturated hydrocarbon group having 3 to 7 carbon atoms and a single carbon-carbon double bond, provided that the double bond cannot be on the α-carbon atoms. Typical alkenyl groups include, for example, 2-propenyl, 2-butenyl, 3-butenyl, and the like.

The term "lower alkynyl" refers to an unsaturated hydrocarbon group having from 3 to 7 carbon atoms, and a single carbon-carbon triple bond, provided that the triple bond cannot be on the α-carbon atom. Typical alkynyl groups include, for example, 2-propynyl, 2-butynyl, 3-butynyl, and the like.

The term "alkoxy" refers to the group having the formula RO- wherein R is a lower alkyl as defined above. Typical alkoxy groups include, for example, methoxy, ethoxy, t-butoxy, and the like.

The term "aryl-alkyl" refers to an aryl substituted alkyl group, such as, for example, benzyl or phenylethyl.

The broadest aspect of the present invention is the group of compositions comprising

rafoxanide;

a solvent selected from dimethyl isosorbide and glycofurol;

optionally a nonionic surfactant; and

optionally a compound of formula I

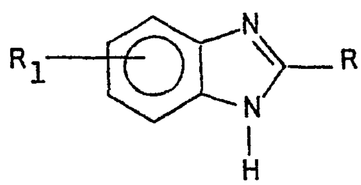

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O,

S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

One preferred subgenus of compositions of the invention is the anthelmintic benzimidazole-free composition comprising rafoxanide and a solvent selected from dimethyl isosorbide and glycofurol.

Another preferred subgenus of compositions of the invention is the anthelmintic benzimidazole-free composition comprising rafoxanide, a nonionic surfactant, and a solvent selected from dimethyl isosorbide and glycofurol.

Another preferred subgenus of compositions of the invention is that wherein the anthelmintic benzimidazole derivative is a compound of formula I

wherein

R is $-NHCOOR_2$, where $R_2$ is lower alkyl;

$R_1$ is $-S(O)_mR_3$, -SCN, $-OR_3$, or $-Y_1(CH_2)_nY_2R_4$, where

$Y_1$ and $Y_2$ are each independently O, S, S(O), or $S(O)_2$;

$R_4$ is lower alkyl, phenyl, or naphthyl;

n is an integer from 1 to 4;

$R_3$ is lower alkyl, cycloalkyl having 3 to 7 carbon atoms, lower alkenyl or lower alkynyl, phenyl, benzyl, phenylethyl, or naphthyl; and

m is 1 or 2.

A preferred class is that wherein $R_1$ is $-S(O)_mR_3$, especially where m is 1. Preferred species are those wherein $R_3$ is ethyl, 1-propyl, 2-butyl, or phenyl, especially 1-propyl or phenyl. Another preferred class is that wherein $R_1$ is $-S(O)_mR_3$ where m is 2. A preferred species is that wherein $R_3$ is phenyl. Another preferred class is that wherein $R_1$ is $-Y_1(CH_2)_nY_2R_4$, especially where $Y_1$ is S(O) and $Y_2$ is O. Preferred species are those wherein $R_4$ is methyl, especially where n is 1 or 2. A preferred species of the invention is that wherein the anthelmintic benzimidazole derivative is oxfendazole.

Another preferred subgenus of compositions of the invention is that wherein the anthelmintic benzimidazole derivative is a compound of formula I

or a pharmaceutically acceptable salt thereof, wherein

R is $-NHCOOR_2$ or 4-thiazolyl, where $R_2$ is alkyl, or aryl; and

$R_1$ is H, $R_3$, or $-XR_3$, where

X is O, S, -C(O)-, or -NHCOO-; and

$R_3$ is alkyl or cycloalkyl having 3 to 7 carbon atoms, aryl or aryl-alkyl, optionally substituted with halo, alkyl, hydroxy, or alkoxy.

Examples of this subgenus include fenbendazole, albendazole, mebendazole, oxibendazole, parbendazole, flubendazole, thiabendazole, cyclobendazole, or cambendazole. A preferred subclass of the invention is that wherein the anthelmintic benzimidazole is fenbendazole, mebendazole, cambendazole or albendazole. A preferred species of the invention is that wherein the anthelmintic benzimidazole derivative is albendazole.

A particularly preferred subgenus is that wherein rafoxanide is present in a concentration between 20 and 4500 g per liter and the anthelmintic benzimidazole derivative is present in a therapeutically effective amount.

Another particularly preferred subgenus is that wherein rafoxanide is present in a concentration between 20 and 4500 g per liter, the nonionic surfactant is present in a concentration between 20 and 4500 g per liter and the anthelmintic benzimidazole derivative is present in a therapeutically effective amount.

Another aspect of the invention is a method for treating helminth and fluke infections in a ruminant by administering a therapeutically effective amount of one of the compositions described above.

Compositions of the invention may be tested by direct efficacy tests (wherein test animals are infected, administered the compound of the invention or neutral solvent, and necropsied to check for remaining parasites) or by blood plasma level demonstrations where the composition comprises individual compounds of known efficacy.

## ADMINISTRATION

These compositions are effective when administered orally. It is preferred to administer the compositions of the invention intraruminally.

In view of the foregoing as well as in consideration of the degree of severity of the condition being treated, age of subject and so forth, all of which factors are determinable by routine experimentation by one skilled in the art, the effective dosage in accordance herewith can vary over a wide range. The following discussion will be in terms of oxfendazole, however, other anthelmintics such as those defined above can be substituted. Dosage

ranges for each commercially available compound are known in the art; ranges for compounds not commercially available are similar. Generally, a therapeutically effective amount ranges from about 0.1 to about 50 mg/kg body weight per day for oxfendazole and from about 0.2 to about 45 mg/kg body weight per day for rafoxanide. Other anthelmintic benzimidazole derivatives may have varying dosage ranges. Preferably, oxfendazole and rafoxanide are administered intraruminally to cattle in dosages of about 2.5 to 4.5 mg/kg and about 5.0 mg/kg body weight per day, respectively. In other terms, for a 100 kg subject, a therapeutically effective amount in accordance herewith would be, in preferred embodiments from about 10 mg to about 5000 mg of oxfendazole per day per subject and from about 20 mg to about 4500 mg of rafoxanide per day per subject, and preferably about 250 to 450 mg and 500 mg, respectively, per day per subject.
When the compositions of the invention are administered by rumen injector, the dose range may vary from about 0.1 ml per 50 kg body weight to about 20 ml per 50 kg body weight, preferably about 1.0 ml per 50 kg. The maximum dose by single injection using rumen injectors presently commercially available is about 20 ml, but it is preferred to administer about 10 ml per dose per subject. The preferred embodiments of the invention contain from about 5 mg/ml oxfendazole or similar anthelmintic benzimidazole derivative and from about 10 mg/ml rafoxanide to about 500 mg/ml oxfendazole or similar anthelmintic benzimidazole derivative to about 400 mg/ml rafoxanide, and preferably about 125 to 225 mg/ml oxfendazole or similar anthelmintic benzimidazole derivative and 500 mg/ml rafoxanide.

If a nonionic surfactant such as Triton®X100 is used in the formulation, a therapeutically effective amount generally ranges from about 0.1 to about 50 mg/kg

body weight per day for oxfendazole, from about 0.2 to about 45 mg/kg body weight per day for rafoxanide, and from about 0.2 to about 45 mg/kg body weight per day for the nonionic surfactant (Triton®X100). Other anthelmintic benzimidazole derivatives may have varying dosage ranges. Preferably, oxfendazole, rafoxanide, and Triton®X100 are administered intraruminally to cattle in dosages of about 2.5 to 4.5 mg/kg, about 5.0 mg/kg body weight per day, and about 5.0 mg/kg body weight per day, respectively. In other terms, for a 100 kg subject, a therapeutically effective amount in accordance herewith would be, in preferred embodiments from about 10 mg to about 5000 mg of oxfendazole per day per subject, from about 20 mg to about 4500 mg of rafoxanide per day per subject, and from about 20 mg to about 4500 mg of Triton®X100 per day per subject, and preferably about 250 to 450 mg, 500 mg, and 500 mg, respectively, per day per subject.

When the compositions of the invention, including a nonionic surfactant, are administered by rumen injector, the dose range may vary from about 0.1 ml per 50 kg body weight to about 20 ml per 50 kg body weight, preferably about 1.0 ml per 50 kg. The maximum dose by single injection using rumen injectors presently commercially available is about 20 ml, but it is preferred to administer about 10 ml per dose per subject. The preferred embodiments of the invention contain from about 5 mg/ml oxfendazole or similar anthelmintic benzimidazole derivative, from about 10 mg/ml rafoxanide, and from about 10 mg/ml Triton®X100, to about 500 mg/ml oxfendazole or similar anthelmintic benzimidazole derivative, to about 400 mg/ml rafoxanide, and to about 400 mg/ml Triton®X100, and preferably about 125 to 225 mg/ml oxfendazole or similar anthelmintic benzimidazole derivative, 250 mg/ml rafoxanide, and 250 mg/ml Triton®X100.

Preferably, oxfendazole and rafoxanide are administered orally to sheep in dosages of about 5.0 mg/kg and about 7.5 mg/kg body weight per day, respectively. In other terms, for a 30 kg subject, a therapeutically effective amount in accordance herewith would be, in preferred embodiments from about 3 mg to about 1500 mg of oxfendazole per day per subject and from about 6 mg to about 1500 mg of rafoxanide per day per subject, and preferably about 125 to 150 mg and 225 mg, respectively, per day per subject.

Preferably, oxfendazole, rafoxanide, and Triton®X100 are administered orally to sheep in dosages of about 2.5 to 4.5 mg/kg, about 7.5 mg/kg body weight per day, and about 7.5 mg/kg body weight per day, respectively. In other terms, for a 30 kg subject, a therapeutically effective amount in accordance herewith would be, in preferred embodiments from about 3 mg to about 1500 mg of oxfendazole per day per subject, from about 6 mg to about 1500 mg of rafoxanide per day per subject, and from about 6 mg to about 1500 mg of Triton®X100 per day per subject, and preferably about 136 to 150 mg, 225 mg, and 225 mg, respectively, per day per subject.

## PREPARATION OF THE INVENTION

Rafoxanide, oxfendazole (and similar anthelmintic benzimidazole derivatives), dimethyl isosorbide, and glycofurol and Triton®X100 are each available from commercial sources. Alternatively, rafoxanide may be made by the process described in U.S. Pat. No. 3,914,418, which is incorporated herein by reference. Oxfendazole and similar anthelmintic benzimidazole derivatives may be made by the process described in U.S. Pat. No. 3,929,821, which is incorporated herein by reference.

Albendazole and similar anthelmintic benzimidazole derivatives may be made by the process described in U.S. Patent No. 3,915,986, which is incorporated herein by reference. Fenbendazole and similar anthelmintic benzimidazole derivatives may be made by the process described in Belgian Patent No. 793,358. Parbendazole and similar anthelmintic benzimidazole derivatives may be made by the processes described in U.S. Patent Nos. 3,480,642 and 3,574,845 which are incorporated herein by reference. Oxibendazole and similar anthelmintic benzimidazole derivatives may be made by the process described in U.S. Patent No. 3,574,845, which is incorporated herein by reference. Flubendazole, cyclobendazole, and similar anthelmintic benzimidazole derivatives may be made by the process described in U.S. Patent No. 3,657,267, which is incorporated herein by reference. Cambendazole and similar anthelmintic benzimidazole derivatives may be made by the process described in South African Patent No. 6,800,351. Mebendazole and similar anthelmintic benzimidazole derivatives may be made by the process described in U.S. Patent No. 3,657,267, which is incorporated herein by reference.

To prepare the composition of the invention, rafoxanide is dissolved in a quantity of the solvent with stirring. When glycofurol is used as the solvent, it is preferred to heat the solvent to about 45°C to facilitate formation of the solution. When dimethyl isosorbide is used, no heating is necessary. The Triton X100, where desired, is then added and the solution mixed. The anthelmintic benzimidazole derivative, where desired, is then suspended in the solution, preferably using a homogenizer, and the solution diluted to full volume with more solvent.

All of the examples set forth below are for oxfendazole. However, other benzimidazoles of formula I above can be substituted for oxfendazole.

EXAMPLE 1

Rafoxanide, 250 g, was dissolved in 750 mL of glycofurol at 45°C with stirring. Then, 125 g of oxfendazole was suspended in the solution using a high speed homogenizer. The suspension was then brought up to full volume by adding glycofurol to make 1 liter. The composition was then administered in 1 to 20 mL doses.

In a similar manner a concentration of 225 mg/ml oxfendazole can be prepared.

EXAMPLE 2

Rafoxanide, 250 g, was dissolved in 750 mL of dimethyl isosorbide at 25°C with stirring. Then, 125 g of oxfendazole was suspended in the solution using a high speed homogenizer. The suspension was then brought up to full volume by adding dimethyl isosorbide to make 1 liter. The composition was then administered in 1 to 20 mL doses.

In a similar manner a concentration of 225 mg/ml oxfendazole can be prepared.

EXAMPLE 3

Rafoxanide, 250 g, was dissolved in 750 mL of glycofurol at 45°C with stirring. Then 250 g of Triton®X100 was added and the solution mixed. Then, 125 g of oxfendazole was suspended in the solution using a high speed homogenizer. The suspension was then brought up to full volume by adding glycofurol to make 1 liter. The composition was then administered in 1 to 20 mL doses.

In a similar manner a concentration of 225 mg/ml oxfendazole can be prepared.

EXAMPLE 4

Rafoxanide, 250 g, was dissolved in 750 mL of dimethyl isosorbide at 25°C with stirring. Then 250 g of Triton®X100 was added and the solution mixed. Then, 125 g of oxfendazole was suspended in the solution using a high speed homogenizer. The suspension was then brought up to full volume by adding dimethyl isosorbide to make 1 liter. The composition was then administered in 1 to 20 mL doses.

In a similar manner a concentration of 225 mg/ml oxfendazole can be prepared.

EXAMPLE 5

Rafoxanide, 250 g, was dissolved in 750 mL of glycofurol at 45°C with stirring. The solution was then brought up to full volume by adding glycofurol to make 1 liter. The composition was then administered in 1 to 20 mL doses.

EXAMPLE 6

Rafoxanide, 250 g, was dissolved in 750 mL of dimethyl isosorbide at 25°C with stirring. The solution was then brought up to full volume by adding dimethyl isosorbide to make 1 liter. The composition was then administered in 1 to 20 mL doses.

EXAMPLE 7

Rafoxanide, 250 g, was dissolved in 750 mL of glycofurol at 45°C with stirring. Then 250 g of Triton®X100 was added and the solution mixed. The solution was then brought up to full volume by adding glycofurol to make 1 liter. The composition was then administered in 1 to 20 mL doses.

## EXAMPLE 8

Rafoxanide, 250 g, was dissolved in 750 mL of dimethyl isosorbide at 25°C with stirring. Then 250 g of Triton®X100 was added and the solution mixed. The solution was then brought up to full volume by adding dimethyl isosorbide to make 1 liter. The composition was then administered in 1 to 20 mL doses.

## EXAMPLE 9

Rafoxanide, 150 g, was dissolved in 750 mL of glycofurol at 45°C with stirring. Then, 90.6 g of oxfendazole was suspended in the solution using a high speed homogenizer. The suspension was then brought up to full volume by adding glycofurol to make 1 liter. The composition was then administered in 1 to 20 mL doses.

## EXAMPLE 10

Rafoxanide, 150 g, was dissolved in 750 mL of dimethyl isosorbide at 25°C with stirring. Then, 90.6 g of oxfendazole was suspended in the solution using a high speed homogenizer. The suspension was then brought up to full volume by adding dimethyl isosorbide to make 1 liter. The composition was then administered in 1 to 20 mL doses.

## EXAMPLE 11

Rafoxanide, 150 g, was dissolved in 750 mL of glycofurol at 45°C with stirring. Then 150 g of Triton®X100 was added and the solution mixed. Then, 90.6 g of oxfendazole was suspended in the solution using a high speed homogenizer. The suspension was then brought up to full volume by adding glycofurol to make 1 liter. The composition was then administered in 1 to 20 mL doses.

EXAMPLE 12

Rafoxanide, 150 g, was dissolved in 750 mL of dimethyl isosorbide at 25°C with stirring. Then 150 g of Triton®X100 was added and the solution mixed. Then, 90.6 g of oxfendazole was suspended in the solution using a high speed homogenizer. The suspension was then brought up to full volume by adding dimethyl isosorbide to make 1 liter. The composition was then administered in 10 mL doses.

EXAMPLE 13

The following is an experimental procedure to demonstrate the efficacy of the invention.

Twenty (20) head of cattle, preferably from a single source, known to be infected with nematode parasites as evidenced by positive fecal egg counts are used. The animals would be preferably of one sex, either all steers or all heifers, and about six months of age.. They will have been grazing pastures known to to be contaminated with cattle parasite eggs and larvae. About 150 metacercariae of Fasciola hepatica are administered to each animal.

The animals are treated 10-12 weeks after infection with F. hepatica. The animals were treated with a control, a composition containing oxfendazole and rafoxanide, or a composition containing rafoxanide. Animals are then subjected to necropsy between 7 and 14 days post-treatment. Gastrointestinal tract, lungs, and liver are examined using standard parasitological techniques for nematode and trematode parasites. The compositions of the invention prove to be efficacious in this assay.

EXAMPLE 14

The following is an experimental procedure to demonstrate blood plasma levels of the active compounds administered.

Ten cows were selected for study. Four cows were injected rumenally with a composition containing rafoxanide at the rate of 1 ml per 50 kg body weight, three cows were injected rumenally with a composition containing oxfendazole and rafoxanide at the rate of 1 ml per 50 kg body weight, and three cows were dosed orally with rafoxanide in commercially available form at the rate of 7.5 mg per kg body weight.

Blood samples were drawn and analyzed before administration and on days 1, 2, 3, 7, 10, and 13 following administration. The compositions of the invention show blood plasma levels equal or superior to plasma levels obtained using commercially available formulations.

CLAIMS:

1. A composition comprising the compound rafoxanide;

a solvent selected from dimethyl isosorbide and glycofurol;

optionally a nonionic surfactant; and

optionally a compound of formula I

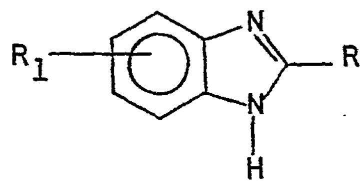

(I)

or a pharmaceutically acceptable salt thereof, wherein

R is $-NHCOOR_2$ or 4-thiazolyl, where $R_2$ is alkyl, or aryl;

$R_1$ is H, $R_3$, $-XR_3$, or $-Y_1(CH_2)_nY_2R_4$, where

X is O, $S(O)_m$, -C(O)-, or -NHCOO-;

$Y_1$ and $Y_2$ are each independently O, S, S(O), or $S(O)_2$;

$R_4$ is lower alkyl, phenyl, or naphthyl;

n is an integer from 1 to 4;

$R_3$ is -CN, alkyl, cycloalkyl having 3 to 7 carbon atoms, lower alkenyl or lower alkynyl, aryl or aryl-alkyl, optionally substituted with halo, alkyl, hydroxy, or alkoxy; and

m is 0, 1, or 2.

2. The composition of claim 1 which includes the compounds of formula I

wherein

R is $-NHCOOR_2$, where $R_2$ is lower alkyl;

$R_1$ is $-S(O)_mR_3$, -SCN, $-OR_3$, or $-Y_1(CH_2)_nY_2R_4$, where

$Y_1$ and $Y_2$ are each independently O, S, S(O), or $S(O)_2$;

$R_4$ is lower alkyl, phenyl, or naphthyl;

n is an integer from 1 to 4;

$R_3$ is lower alkyl, cycloalkyl having 3 to 7 carbon atoms, lower alkenyl or lower alkynyl, phenyl, benzyl, phenylethyl, or naphthyl; and

m is 1 or 2.

3. The composition of claim 2 which includes the compound of formula I, wherein $R_1$ is $-S(O)R_3$.

4. The composition of claim 3 wherein $R_3$ is ethyl, 1-propyl, 2-butyl or phenyl.

5. The composition of claim 4 wherein the compound of formula I is

methyl /5-(1-propylsulfinyl)-1H-benzimidazol-2-yl/-carbamate; methyl /5-(ethylsulfinyl)-1H-benzimidazol-2-yl/-carbamate; or methyl /5-(2-butylsulfinyl)-1H-benzimidazol-2-yl/-carbamate.

6. The composition of claim 5 wherein the compound of formula I is methyl /5-(n-propylsulfinyl)-1H-benzimidazol-2-yl/-carbamate.

7. The composition of claim 4 wherein $R_3$ is phenyl and $R_2$ is methyl, i.e., the compound oxfendazole.

8. The composition of claim 2 which includes a compound of formula I, wherein $R_1$ is $-S(O)_2R_3$.

9. The composition of claim 8 wherein $R_3$ is phenyl, i.e., the compound methyl /5-(phenylsulfonyl)-1H-benzimidazol-2-yl/-carbamate.

10. The composition of claim 2 which includes a compound of formula I, wherein $R_1$ is $-Y_1(CH_2)_nY_2R_4$.

11. The composition of claim 10 wherein $Y_1$ is S(O) and Y is O.

12. The composition of claim 11 wherein $R_4$ is methyl.

13. The composition of claim 12 wherein n is 1, i.e., the compound methyl /5-(methoxymethylsulfinyl)-1H-benzimidazol-2-yl/-carbamate.

14. The composition of claim 12 wherein n is 2, i.e., the compound methyl /5-(2-methoxyethylsulfinyl)-1H-benzimidazol-2-yl/-carbamate.

15. The composition of claim 1 which includes the compounds of formula I

or a pharmaceutically acceptable salt thereof, wherein

R is $-NHCOOR_2$ or 4-thiazolyl, where R is alkyl or aryl; and

$R_1$ is H, $R_3$ or $-XR_3$, where

X is O, S, -C(O)- or -NHCOO-; and

$R_3$ is alkyl or cycloalkyl having 3 to 7 carbon atoms, aryl or aryl-alkyl, optionally substituted with halo, alkyl, hydroxy or alkoxy.

16. The composition of claim 15 wherein the compound of formula I is fenbendazole, albendazole, mebendazole, oxibendazole, parbendazole, flubendazole, thiabenzadole, cyclobendazole or cambendazole.

17. The composition of any one of claims 1 to 16 wherein the compound of formula I is present in a concentration between 75 and 225 mg/ml.

18. The composition of any one of claims 1 to 17 wherein the rafoxanide is present in a concentration between 150 and 500 mg/ml.

19. The composition of any one of claims 1 to 18 wherein the nonionic surfactant is selected from nonoxynol, octoxynol or nonoxinol.

20. The composition of claim 19 wherein the nonionic surfactant is Triton® X100.

21. The use of a composition according to any one of claims 1 to 20 for treating parasitic infestations in a ruminant.

European Patent Office

# EUROPEAN SEARCH REPORT

0202568
Application number

EP 86 10 6403

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | GB-A-2 150 024 (MAY & BAKER LTD.)<br>* Page 1, abstract *<br>--- | 1-21 | A 61 K 31/165<br>A 61 K 31/415<br>A 61 K 47/00 //<br>(A 61 K 31/415<br>A 61 K 31:165) |
| A | US-A-4 076 825 (RUDIGER D. HAUGWITZ)<br>* Column 2, line 57 - column 4, line 20 *<br>----- | 1-21 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 21-08-1986 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document